Europäisches Patentamt

European Patent Office

Office européen des brevets

(11) Veröffentlichungsnummer: **0 322 631 B1**

## (12) EUROPÄISCHE PATENTSCHRIFT

(45) Veröffentlichungstag der Patentschrift:
12.06.91 Patentblatt 91/24

(51) Int. Cl.⁵: **G01N 31/22**, G01N 33/52

(21) Anmeldenummer: 88120784.9

(22) Anmeldetag: 13.12.88

(54) Verfahren und Reagenz zur Bestimmung von Persäuren.

(30) Priorität: 19.12.87 DE 3743224

(43) Veröffentlichungstag der Anmeldung:
05.07.89 Patentblatt 89/27

(45) Bekanntmachung des Hinweises auf die
Patenterteilung:
12.06.91 Patentblatt 91/24

(84) Benannte Vertragsstaaten:
BE CH DE FR GB IT LI NL

(56) Entgegenhaltungen:
EP-A- 0 029 917
US-A- 3 485 588
CHEMICAL ABSTRACTS, Band 109, Nr. 20, 14.
November 1988, Seite 861, Nr. 182821t, Columbus, Ohio, US; D.M. DAVIES et al.:
"Determination of peracids in the presence of
a very large excess of hydrogen peroxide
using a rapid and convenient spectrophotometric method", & ANALYST (LONDON) 1988,
113(9), 1477-9

(56) Entgegenhaltungen:
ANALYTICAL CHEMISTRY, Band 51, Nr. 7,
Juni 1979, Seiten 790-796, American Chemical
Society, Columbus, Ohio, US; L.A. LAZAROU
et al.: "Kinetic study of the iron(II)-induced
perbromate-iodide reaction with an iodide ion
selective electrode and kinetic determination
of iron, perbromate, ethylenediaminetetraacetic acid, diethylenetriaminepentaacetic acid,
and ethylene glycol-bis(2-aminoethyl ether)-N,N,N',N'-tetraacetic acid"
CHEMICAL ABSTRACTS, Band 97. Nr. 20, 15.
November 1982, Seite 772, Nr. 174029j, Columbus, Ohio, US; E.L. DICKSON et al.:
"Determination of molybdenum by the catalyzed peroxyacetic acid-iodide-ascorbic acid reaction", & ANAL. CHIM. ACTA 1982, 139,
117-26

(73) Patentinhaber: MERCK PATENT
GESELLSCHAFT MIT BESCHRÄNKTER
HAFTUNG
Frankfurter Strasse 250 Postfach 4119
W-6100 Darmstadt (DE)

(72) Erfinder: Fischer, Wolfgang, Dr.
Kröhweg 27
W-6100 Darmstadt (DE)
Erfinder: Arlt, Edda
Auf dem Sand 3
W-6109 Nieder-Ramstadt (DE)
Erfinder: Brabänder, Barbara
Odenwaldstrasse 35
W-6105 Ober-Ramstadt (DE)

## Beschreibung

Die Erfindung betrifft ein Verfahren und Reagenz zur Bestimmung von Persäuren, insbesondere zur Bestimmung von Peressigsäure neben Wasserstoffperoxid.

Peressigsäure wird heute wegen der entstehenden umweltfreundlichen Reaktionsprodukte Essigsäure, Wasser und Sauerstoff immer mehr für desinfizierende Zwecke eingesetzt. Da die desinfizierende Wirkung von der Konzentration der Persäuren abhängig ist, die Lösungen sich aber mit der Zeit zersetzen, sind zur Überprüfung der ausreichenden Wirkung immer wieder Gehaltsbestimmungen wichtig.

Das Hauptproblem bei der Gehaltsbestimmung besteht darin, daß wäßrige Peressigsäurelösungen neben Essigsäure auch Wasserstoffperoxid enthalten. Dieses zeigt in den meisten Fällen ähnliche Reaktionen wie die Peressigsäure, so daß man mit den üblichen Verfahren eine Summenbestimmung erhält. Diese Summenbestimmung ist jedoch nicht sehr aussagekräftig, weil die desinfizierende Wirkung vor allem von der Peressigsäure ausgeht.

Quantitative Methoden zur Bestimmung von Persäuren, auch in Gegenwart von Wasserstoffperoxid oder anderen Peroxiden, sind Z.B. in Anal. Chim. Acta 155, 139 (1983) beschrieben. Danach lassen sich Peressigsäure und Wasserstoffperoxid nur als Summe bestimmen. Aus Chemical Abstracts, Vol. 18, 1970, Abstr. 4100, ist ein Verfahren zur photometrischen Bestimmung von Persäuren in Gegenwart von Wasserstoffperoxid bekannt, das darauf beruht, daß organische Persäuren m-Phenylendiamin bei 50-55°C und einem pH-Wert von 2-3 innerhalb von 30-40 Minuten oxidieren, während Wasserstoffperoxid unter diesen Bedingungen nicht oxidierend wirkt. Diese Methode ist ebenso wie die aus zwei nacheinander mit unterschiedlichen Reagenzien durchzuführenden Titrationen sehr zeitaufwendig und verlangt eine geeignete Laborausstattung und eingearbeitetes Laborpersonal.

Der vorliegenden Erfindung liegt die Aufgabe zugrunde, ein Verfahren und Reagenz zur Verfügung zu stellen, mit dem die Bestimmung von Persäuren neben anderen Peroxiden auf einfache Weise durchgeführt werden kann. Überraschenderweise wurde gefunden, daß man ein für Persäuren in Gegenwart von Wasserstoffperoxid spezifisches, einfach durchzuführendes Verfahren erhält, wenn man die üblichen Farbreagenzien und Puffer für den Peroxid-Nachweis einsetzt, dabei jedoch den sauerstoffübertragenden Katalysator (Z.B. Ammoniummolybdat, Peroxidase) wegläßt und dafür ein Jodid zusetzt.

Unter diesen Bedingungen reagiert Wasserstoffperoxid praktisch nicht oder nur sehr stark verzögert, während die Persäure schnell und deutlich reagiert. Ein solches Verfahren gestattet deshalb die Bestimmung von Persäuren in Gegenwart von Wasserstoffperoxid.

Gegenstand der Erfindung ist ein Verfahren zur Bestimmung von Persäuren in Gegenwart von Wasserstoffperoxid, das dadurch gekennzeichnet ist, daß man die Probelösung mit einem Chromogen, einem Jodid und gegebenenfalls einem Puffer enthaltenden Reagenz zusammenbringt und die Farbreaktion spektrophotometrisch oder visuell auswertet.

Ein weiterer Gegenstand der Erfindung ist ein Reagenz zur Bestimmung von Persäuren, enthaltend ein Chromogen, ein Jodid und gegebenenfalls einen Puffer. Das Reagenz liegt vorzugsweise in Form einer imprägnierten Matrix vor. Zur spezifischen Bestimmung von Peressigsäure in Gegenwart von Wasserstoffperoxid ist die Matrix mit einem Chromogen, einem Jodid und einem Puffer imprägniert.

Nach dem erfindungsgemäßen Verfahren lassen sich im Prinzip alle Persäuren bestimmen, vorzugsweise Peressigsäure, Perpropionsäure, Perbuttersäure, Perbenzoesäure und deren Derivate. Der Peressigsäure kommt dabei eine besondere Bedeutung zu, weil diese aufgrund ihrer bakteriziden, fungiziden, sporiziden und viruziden Wirkung in großem Umfang als Desinfektionsmittel angewendet wird. Peressigsäure zeichnet sich gegenüber anderen Desinfektionsmitteln besonders durch ihre kurze Einwirkungszeit und ihre niedrigen Anwendungskonzentrationen aus (0,01-0,2 %). Gerade in verdünnten Lösungen tritt jedoch eine relativ schnelle Hydrolyse zu Essigsäure und Wasserstoffperoxid ein, so daß der Gehalt an Peressigsäure, von der die desinfizierende Wirkung in erster Linie ausgeht, ständig abnimmt.

Als Chromogene können alle in der Literatur bekannten Chromogene eingesetzt werden, die in Gegenwart von Peroxiden oxidierbar sind. Geeignet sind zum Beispiel aromatische Amine wie o-Toluidin, o- und p-Phenylendiamin, 4,4'-Diaminodiphenylamin, 2,7-Diaminofluoren, Benzidin und Benzidinderivate wie o-Tolidin, o-Dianisidin oder Tetramethylbenzidin, Phenole wie o-Kresol, Guajacol, Pyrogallol, Hydrochinon oder 4-Chlor-1-naphthol, Leukofarbstoffe wie Leukomalachitgrün oder reduziertes 2,6-Dichlorphenolindophenol, vorzugsweise Tetramethylbenzidin.

Daneben sind aber auch die bekannten Reagenzkombinationen einsetzbar, die sich von Peroxiden in Gegenwart eines Katalysators zu Farbstoffen oxidieren lassen, wie 4-Aminoantipyrin mit Phenol oder N,N-Dialkylanilin oder auch 3-Methyl-2-benzthiazolinonhydrazon mit N,N-Dialkylanilinen.

Die Chromogene müssen im stöchiometrischen Überschuß vorliegen. Auch bei der höchsten nachzuweisenden Persäurekonzentration sollte noch unverändertes Chromogen vorliegen.

Ein wesentlicher Faktor für die erfindungsgemäße Bestimmung von Persäuren ist der Zusatz von Jodid anstelle eines sauerstoffübertragenden Katalysators, wie er für die üblichen Peroxidnachweise erforderlich ist. Ohne Jodid wäre der erfindungsgemäße Test vor allem als Schnelltest völlig ungeeignet, weil eine Erwärmung auf über 50°C für mehr als eine halbe Stunde erforderlich wäre, bevor der Test ausgewertet werden kann. Geeignete Jodide sind die Alkalijodide und Ammoniumjodid, vorzugsweise Kaliumjodid.

Die Jodidkonzentration kann sehr gering sein, z.B. in der Größenordnung von einem Zehntel der Chromogenkonzentration. Das Jodid wird zwar bei der ablaufenden Redoxreaktion zunächst verbraucht, dann aber auch neu gebildet.

Um die Reaktion in einem optimalen pH-Bereich ablaufen zu lassen, enthält das Reagenz gegebenenfalls auch Puffersubstanzen. Diese sind nicht erforderlich, wenn die Farbreaktion spektrophotometrisch ausgewertet wird. Erfolgt die Bestimmung der Persäuren jedoch mit Hilfe einer mit den erforderlichen Reagenzien imprägnierten Matrix, dann ist die Anwesenheit von Puffersubstanzen von Vorteil.

Als Puffer kommen solche in Frage, die einen pH-Bereich von 3 bis 6 einhalten und die Nachweisreaktion nicht stören. Die einzusetzende Pufferkonzentration richtet sich nach dem pH-Wert der Probelösung und nach der dort eventuell vorliegenden freien Säure bzw. Base. Als Puffer sind die gebräuchlichen Salze wie Phosphate, Citrate, Borate usw. geeignet, vorzugsweise Phosphatpuffer.

Das erfindungsgemäße Reagenz kann sowohl als Lösung als auch in Form von damit imprägnierten saugfähigen Matrices angewendet werden, vorzugsweise in Form von Teststäbchen.

Als saugfähige Matrices können alle verwendet werden, die üblicherweise für solche Schnelltests im Gebrauch sind. Am weitesten verbreitet ist die Verwendung von Filterpapier, jedoch können auch andere saugfähige Cellulose- oder Kunststoffprodukte eingesetzt werden. Die saugfähigen Träger, vorzugsweise Filterpapier, werden in an sich bekannter Weise mit Tränklösungen imprägniert, die die zur Bestimmung der Persäure notwendigen Reagenzien enthalten. Die getränkten und getrockneten Papiere können zu quadratischen bzw. rechteckigen Zonen verarbeitet werden, die ihrerseits in bekannter Weise auf Kunststoffolien, Papier- oder Metallstreifen aufgeklebt bzw. aufgesiegelt werden können.

Die saugfähigen Träger können auch vor dem Imprägnieren in Streifenform auf ein Kunststoffband aufgebracht und nach dem Imprägnieren senkrecht zur Streifenrichtung in handliche Stäbchen geschnitten werden.

Zur Durchführung des Tests wird der saugfähige Träger etwa 1 bis 3 Sekunden in die zu untersuchende Lösung getaucht und nach etwa 15 Sekunden wird die Färbung mit einer Farbskala verglichen und der entsprechende Gehalt an Persäure abgelesen.

Zur quantitativen Bestimmung wird eine Reagenzlösung zu der zu untersuchenden Probe in eine Küvette gegeben, gemischt und nach etwa 2 Minuten in einem Spektrophotometer gemessen.

Beispiel 1

Bestimmung von Peressigsäure mit einem Teststäbchen

Ein Filterpapier (Schleicher & Schüll 2992) wird nacheinander mit den folgenden Reagenzlösungen getränkt und nach jeder Tränkung mit warmer Luft getrocknet :

```
Lösung 1:  125    g Natriumdihydrogenphosphat
           125    g Dinatriumhydrogenphosphat
             0,5  g Kaliumjodid
           in 1 l Wasser


Lösung 2:    5    g Tetramethylbenzidin
           in 1 l Ethanol
```

Zur Ermittlung des Nachweisbereichs wird eine 5%ige Peressigsäurelösung verdünnt, um Lösungen im Konzentrationsbereich von 0,001 bis 0,2% zu erhalten. Die genauen Peressigsäure-Konzentrationen der einzelnen Verdünnungen werden nach dem in der Literatur beschriebenen Titrationsverfahren ermittelt.

In diese Lösungen werden Teststäbchen eingetaucht, die durch Aufkleben des imprägnierten Filterpapiers auf weiße Folien hergestellt werden. In Abhängigkeit von der Konzentration entstehen unterschiedlich intensive Blaufärbungen. Wasserstoffperoxidlösungen reagieren nicht.

Die Versuche zeigen, daß folgende Konzentrationen von Peressigsäure unterschiedliche Färbungen erzeugen und sich dadurch mit Hilfe einer entsprechenden Farbskala bestimmen lassen :
0, 5, 10, 30, 60, 100, 500 und 2000 ppm.

Beispiel 2

Je 10 ml Peressigsäurelösungen im Konzentrationsbereich von 0,0005 bis 0,01% werden mit 1 ml einer Lösung von 0,3 g 4-Chlor-1-naphthol und 0,03 g Natriumjodid in 100 ml 1%iger Essigsäure versetzt. Die erhaltenen Lösungen werden in eine 10-mm-Küvette gefüllt, und nach 2 Minuten wird die Extinktion im Spektralphotometer bei 528 nm bestimmt.

Die Abhängigkeit der Extinktion von der Konzentration der Peressigsäure wird graphisch in Form einer Eichkurve dargestellt. Mit Hilfe dieser Eichkurve läßt sich der Peressigsäuregehalt unbekannter Lösungen bestimmen.

## Ansprüche

1. Verfahren zur Bestimmung von Persäuren, in Gegenwart von Wasserstoffperoxid dadurch gekennzeichnet, daß man die Probelösung mit einem Chromogen, einem Jodid und gegebenenfalls einem Puffer enthaltenden Reagenz zusammenbringt und die Farbreaktion spektrophotometrisch oder visuell auswertet.

2. Reagenz zur Bestimmung von Persäuren, in Gegenwart von Wasserstoffperoxid enthaltend ein Chromogen, ein Jodid und gegebenenfalls einen Puffer.

3. Reagenz nach Anspruch 2, dadurch gekennzeichnet, daß es in Form einer imprägnierten Matrix vorliegt.

4. Reagenz zur spezifischen Bestimmung von Peressigsäure in Gegenwart von Wasserstoffperoxid, dadurch gekennzeichnet, daß die Matrix mit einem Chromogen, einem Jodid und einem Puffer imprägniert ist.

5. Reagenz nach Anspruch 4, dadurch gekennzeichnet, daß die Matrix mit Tetramethylbenzidin, Kaliumjodid und einem Phosphatpuffer imprägniert ist.

## Claims

1. Method for determination of peracids in the presence of hydrogen peroxide, characterized in that the sample solution is mixed with a chromogen, an iodide and, where appropriate, a buffer-containing reagent, and the colour reaction is evaluated by spectrophotometry or visually.

2. Reagent for the determination of peracids in the presence of hydrogen peroxide, containing a chromogen, an iodide and, where appropriate, a buffer.

3. Reagent according to Claim 2, characterized in that it is in the form of an impregnated matrix.

4. Reagent for the specific determination of peracetic acid in the presence of hydrogen peroxide, characterized in that the matrix is impregnated with a chromogen, an iodide and a buffer.

5. Reagent according to Claim 4, characterized in that the matrix is impregnated with tetramethylbenzidine, potassium iodide and a phosphate buffer.

## Revendications

1. Procédé pour le dosage des peracides en présence d'eau oxygénée, caractérisé en ce que l'on met en contact la solution prélevée avec un chromogène, un iodure et un réactif contenant éventuellement un tampon et en ce que l'on évalue spectrophotométriquement ou visuellement la réaction colorée.

2. Réactif pour le dosage des peracides en présence d'eau oxygénée, caractérisé en ce qu'il contient un chromogène, un iodure et éventuellement un tampon.

3. Réactif selon la revendication 2, caractérisé en ce qu'il se présente sous la forme d'une matrice imprégnée.

4. Réactif pour le dosage spécifique de l'acide peracétique en présence d'eau oxygénée, caractérisé en ce que la matrice est imprégnée d'un chromogène, d'un iodure et d'un tampon.

5. Réactif selon la revendication 4, caractérisé en ce que la matrice est imprégnée de tétraméthylbenzidine, de iodure de potassium et d'un tampon phosphate.